# EUROPEAN PATENT SPECIFICATION

(11) **EP 4 119 246 B1**
(45) Date of publication and mention of the grant of the patent: **13.09.2023**
(21) Application number: 22184019.2
(22) Date of filing: 11.07.2022
(51) Int. Cl.: B09B 3/38, B09B 101/67, B29L 31/48

(54) **USED PAPER DIAPER PROCESSING APPARATUS**
VORRICHTUNG ZUR VERARBEITUNG VON GEBRAUCHTEN PAPIERWINDELN
APPAREIL DE TRAITEMENT DE COUCHES USAGÉES EN PAPIER

(30) Priority: 16.07.2021 JP 2021118017
(43) Date of publication of application: 18.01.2023
(73) Proprietor: Panasonic Intellectual Property Management Co., Ltd., Osaka-shi, Osaka 540-6207 (JP)
(72) Inventor: MATSUDA, Genichiro, Osaka, 540-6207 (JP); KATAOKA, Hidenao, Osaka, 540-6207 (JP); HINO, Naofumi, Osaka, 540-6207 (JP)
(74) Representative: Eisenführ Speiser

(56) References cited:
- EP-A1- 1 415 733

## Description

### Background of the Invention

### Field of the Invention

The present disclosure relates to a used paper diaper processing apparatus that reduces weight by removing water from a superabsorbent polymer contained in a used paper diaper.

More specifically, the present disclosure relates to a used paper diaper processing apparatus that reduces weight by mixing a superabsorbent polymer that has absorbed water contained in excrement with a chemical containing divalent metal ions, or the like, to lower a water absorption function of the superabsorbent polymer to remove water, and after reducing an amount of water contained in a used paper diaper, performing dehydration.

### Description of the Related Art

A used paper diaper contains a large amount of water, and thus, weight of the used paper diaper may be an issue in a case where a large amount of used paper diapers is handled in nursing care facilities, or the like. The paper diaper uses a surface material sheet that transmits water on an inner side thereof in contact with the body and uses a waterproof material sheet that does not transmit water on an outer side thereof. A portion between the surface material sheet and the waterproof material sheet is filled with pulp and a superabsorbent polymer as a water absorbent. The superabsorbent polymer can retain a large amount of water, and water once absorbed by the superabsorbent polymer is difficult to be removed as it is.

Thus, there has been proposed a used paper diaper processing apparatus that reduces weight by stirring a used paper diaper together with a chemical containing divalent metal ions, or the like, to lower a water absorption function of a superabsorbent polymer to remove water and then performing dehydration processing to reduce an amount of water contained in the used paper diaper (see Patent Document 1).

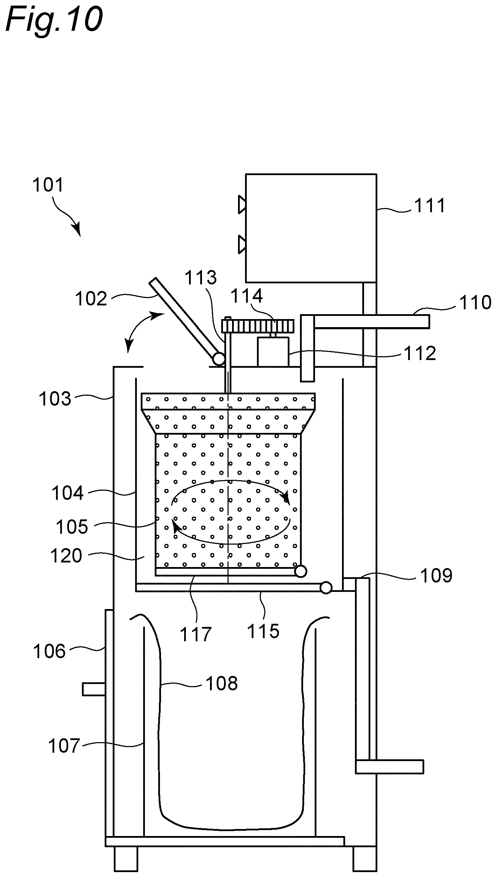

Fig. 10 is a side view of a conventional used paper diaper processing apparatus 101 described in Patent Document 1. The conventional used paper diaper processing apparatus 101 of Fig. 10 includes a processing tank 120, a liquid supply unit 110, a liquid discharge unit 109, a take-out unit 106, a put-into door 102, an apparatus main body 103, a storage container 107, a disposal bag 108, a control apparatus 111, a shaft 113, and a belt 114. The processing tank 120 includes an inner tank 105 and an outer tank 104. Further, an outer tank bottom portion 115 and an inner tank bottom portion 117 that can be opened and closed are provided in a lower part of the processing tank 120.

Next, operation of the conventional used paper diaper processing apparatus 101 will be described.

Water is poured into the outer tank 104 from the liquid supply unit 110, and a certain amount of water is stored in the outer tank 104.

Next, a chemical that lowers a water absorption function of the superabsorbent polymer is poured into the outer tank 104 from the liquid supply unit 110 and mixed with water already poured into the outer tank 104, and a processing liquid is stored in the outer tank 104. As the chemical, a chemical containing divalent metal ions such as calcium chloride is preferably used.

Next, the used paper diaper is put into the inner tank 105 from the put-into door 102, and a motor 112 rotates under the control of the control apparatus 111, so that the shaft 113 rotates via the belt 114. The inner tank 105 connected to the shaft 113 rotates, and stirring processing is executed in the processing tank 120. When the stirring operation is continued for about 10 minutes to 30 minutes, the superabsorbent polymer of the used paper diaper reacts with, for example, divalent metal ions in the chemical, so that water contained in the superabsorbent polymer is removed.

Next, after the liquid such as the processing liquid accumulated in the outer tank 104 is discharged from the liquid discharge unit 109, the inner tank 105 is rotated at high speed with respect to the outer tank 104 to dehydrate the used paper diaper and reduce weight of the used paper diaper. When the outer tank bottom portion 115 and the inner tank bottom portion 117 are opened in a state where the dehydration is completed, the used paper diaper falls into the disposal bag 108 provided in the storage container 107.

In this manner, it is possible to store the used paper diapers whose weight is reduced in the disposal bag 108 without bothering the worker, so that it is possible to reduce work load and hygiene load of the worker.

### Prior Art Document

### Patent Document

Patent Document 1: JP 2020-131157 A
Patent Document 2: EP 1 415 733 A1

However, in the used paper diaper processing apparatus 101 described in Patent Document 1, it is necessary to perform stirring operation for about 10 minutes to 30 minutes to remove water contained in the used paper diaper, and once weight reduction processing is started, the used paper diaper cannot be put into the processing apparatus 101 in the middle. In addition, the worker cannot necessarily perform work of putting-into diapers when the weight reduction processing is completed, and thus, a non-operating time (that is, standby time) occurs until the next diaper weight reduction processing is started, which causes an issue that a processing amount is limited.

Patent Document 2 discloses the preamble of claim 1.

### Summary of the Invention

In view of such a point, one non-limiting and exemplary embodiment provides a used paper diaper processing apparatus capable of holding a used paper diaper in an apparatus even in the middle of weight reduction processing and capable of starting the next weight reduction processing by putting-into an unprocessed used paper diaper every time the weight reduction processing is completed, so that a standby time is eliminated and weight reduction processing can be performed on many diapers with one apparatus.

In other words, the non-limiting and exemplary embodiment provides a used paper diaper processing apparatus capable of performing weight reduction processing on many used paper diapers with one apparatus by eliminating the standby time of the used paper diaper processing apparatus.

Additional benefits and advantages of the disclosed embodiments will be apparent from the specification and Figures. The benefits and/or advantages may be individually provided by the various embodiments and features of the specification and drawings disclosure, and need not all be provided in order to obtain one or more of the same.

In one general aspect, the techniques disclosed here feature: a used paper diaper processing apparatus comprising:
a processing tank that processes a used paper diaper;
a liquid supply unit that supplies a processing liquid to the processing tank;
a liquid discharge unit that discharges the processing liquid from the processing tank;
a take-out unit that takes out and holds a paper diaper processed in the processing tank below the processing tank;
the processing tank including, on at least one of a side surface and a bottom surface, a cylindrical inner tank having a plurality of through holes smaller than the paper diaper, and an outer tank surrounding the side surface and the bottom surface of the inner tank;
a rotation drive unit that rotates the inner tank around an axis of the inner tank;
an opening/closing unit that discharges the paper diaper processed in the processing tank from a bottom portion of the processing tank to the take-out unit; and
a continuous put-into unit that includes in an upper portion of the processing tank, the continuous put-into unit including a plurality of holding units each capable of holding a used paper diaper, and a holding unit rotation drive unit that rotationally drives each of the holding units at a put-into position above an opening connected to the processing tank and a standby position other than the put-into position,
wherein when the holding unit is rotated by the holding unit rotation drive unit and positioned at the put-into position, the used paper diaper held by the holding unit is put into the processing tank from the opening.

According to the used paper diaper processing apparatus according to the above aspect of the present disclosure, even when the used paper diaper processing apparatus is in operation, a used paper diaper collected by the worker is held in the holding unit of the continuous put-into unit, so that the used paper diaper can be held in the apparatus even in the middle of the weight reduction processing, and as soon as the weight reduction processing is completed, the used paper diaper held in the holding unit can be put into the processing tank and the next weight reduction processing can be started, so that the non-operating time of the apparatus can be eliminated. In addition, the worker does not need to start the next weight reduction processing at a timing when the weight reduction processing is completed, so that it is also possible to reduce load on the worker for operating the apparatus. In other words, it is possible to efficiently perform the weight reduction processing of the used paper diaper without bothering the worker.

### Brief Description of the Drawings

These and other aspects and features of the present disclosure will become clear from the following description taken in conjunction with the embodiments thereof with reference to the accompanying drawings, in which:
Fig. 1A is a side cross-sectional view of a used paper diaper processing apparatus according to a first embodiment of the present disclosure;
Fig. 1B is a top view of a cylindrical portion in a state in which a direct put-into door and a storage put-into door are omitted in the used paper diaper processing apparatus in Fig. 1A;
Fig. 2 is a flowchart illustrating a used paper diaper processing method according to the first embodiment of the present disclosure;
Fig. 3 is a side cross-sectional view illustrating a state in which a used paper diaper is placed in a processing tank and stirring processing is performed in the used paper diaper processing apparatus according to the first embodiment of the present disclosure;
Fig. 4 is a side cross-sectional view illustrating a state in which an inner tank is rotated at high speed to perform dehydration processing in the used paper diaper processing apparatus according to the first embodiment of the present disclosure;
Fig. 5 is a side cross-sectional view illustrating a state in which a bottom portion of the processing tank is opened and operation of taking out the used paper diaper after the processing is performed in the used paper diaper processing apparatus according to the first embodiment of the present disclosure;
Fig. 6A is an apparatus side view of a used paper diaper processing apparatus according to a second embodiment of the present disclosure;
Fig. 6B is a top view of a cylindrical portion and a cylindrical portion storage case in a state where a direct put-into door and a storage put-into door of the used paper diaper processing apparatus in Fig. 6A are omitted;
Fig. 6C is a cross-sectional view taken along a line A-A' of Fig. 6B;
Fig. 7A is an enlarged view of a continuous put-into unit of a used paper diaper processing apparatus according to a third embodiment of the present disclosure and is a view illustrating a state in which a removable container is disposed in an upper portion of a put-into slot;
Fig. 7B is a view illustrating a state in which the removable container is provided on a table;
Fig. 8 is a side cross-sectional view illustrating a configuration of a used paper diaper processing apparatus according to a fourth embodiment of the present disclosure;
Fig. 9 is a side cross-sectional view illustrating a state in which a bottom portion of the processing tank is opened and a scraping portion is made to extend to perform scraping operation of the used paper diaper after the processing in the used paper diaper processing apparatus according to the fourth embodiment of the present disclosure; and
Fig. 10 is a cross-sectional view of a conventional used paper diaper processing apparatus liquid processing apparatus.

### Detailed Description

### [First Embodiment]

Hereinafter, a used paper diaper processing apparatus 1 according to embodiments of the present disclosure will be described in detail with reference to the drawings. The same or corresponding parts in the drawings are denoted by the same reference numerals, and description thereof will not be repeated. Note that, for easy understanding of description, in the drawings to be referred to below, configurations are illustrated in a simplified or schematic manner, or some components are omitted. In addition, a dimensional ratio between the components illustrated in each drawing does not necessarily indicate an actual dimensional ratio.

### [Overall Configuration]

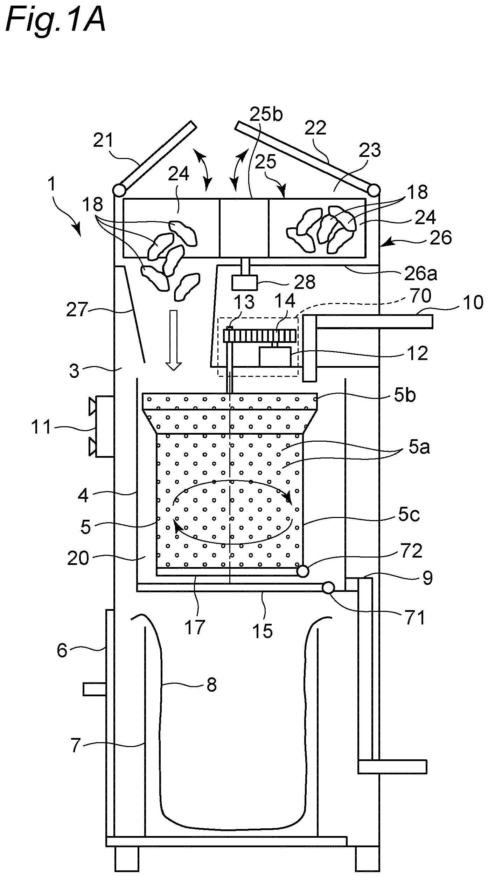

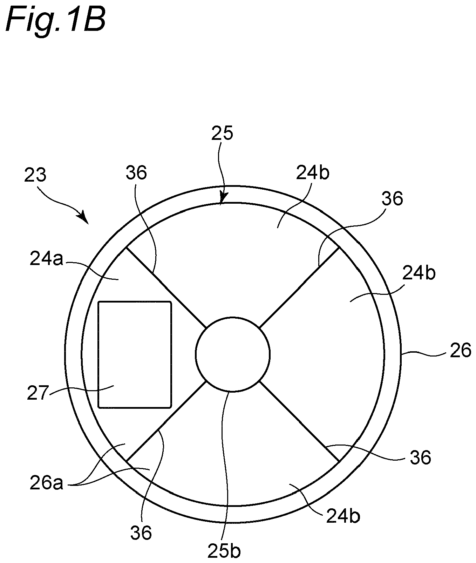

First, an overall configuration of the used paper diaper processing apparatus 1 will be described. Figs. 1A and 1B illustrate a configuration of the used paper diaper processing apparatus 1 according to a first embodiment of the present disclosure. Specifically, Fig. 1A is a side cross-sectional view of the used paper diaper processing apparatus 1, and Fig. 1B is a top view of a cylindrical portion 25 in a state where a direct put-into door 21 and a storage put-into door 22 are omitted in the used paper diaper processing apparatus 1.

The used paper diaper processing apparatus 1 includes at least a processing tank 20, a liquid supply unit 10, a liquid discharge unit 9, a take-out unit 6, and a continuous put-into unit 23. More specifically, the used paper diaper processing apparatus 1 further includes the direct put-into door 21, the storage put-into door 22, an apparatus main body 3, a storage container 7, a disposal bag 8, a control apparatus 11, and a rotation drive unit 70.

The processing tank 20 for performing weight reduction processing on the used paper diaper is disposed in an upper portion in the apparatus main body 3 and includes, for example, a circular cylindrical outer tank 4 and a circular cylindrical inner tank 5 capable of storing a used paper diaper 18, for example. The inner tank 5 is disposed in the outer tank 4.

An upper portion of the inner tank 5 is an upper conical portion 5b constituted as a wide mouth portion in order to easily input the used paper diaper 18, and an intermediate portion is constituted with a cylindrical portion 5c following the upper conical portion 5b.

The outer tank 4 is constituted with a cylinder coaxial with the inner tank 5.

The continuous put-into unit 23 includes in an upper portion of the processing tank 20, at least a plurality of holding units 24 capable of respectively holding the used paper diapers 18, and a holding unit rotation drive unit 28 that rotationally drives each of the holding units 24 at a put-into position I above an opening 27 connected to the processing tank 20 and other standby positions II. Specifically, the continuous put-into unit 23 includes a cylindrical portion 25 having put-into slots (input slots) 24 functioning as an example of the holding unit, a cylindrical portion storage case 26, the opening 27, and a cylindrical portion rotation drive unit 28 functioning as the holding unit rotation drive unit 28.

The cylindrical portion 25 has no bottom surface, and a plurality of put-into slots 24 in each of which a plurality of used paper diapers 18 are stored are arranged while being partitioned from each other in a circumferential direction thereof.

The cylindrical portion storage case 26 is a cylindrical member fixed to an upper end of the apparatus main body 3 and has a bottom surface 26a at a portion other than the opening 27, and the cylindrical portion 25 is disposed inside.

Fig. 1B is a top view of the cylindrical portion storage case 26 and the cylindrical portion 25. A cylindrical portion of the cylindrical portion 25 is open at an upper portion thereof and a lower portion thereof, and in this example, four put-into slots 24 are arranged by being separated by partition plates 36. For convenience, the put-into slot 24 located at the put-into position I overlapping the opening 27 is referred to as a direct put-into slot 24a, and other put-into slots 24 located at the standby positions II other than the put-into position I overlapping the opening 27 are referred to as standby slots 24b.

Note that the put-into slot 24 is not limited to one divided by the partition plate 36, and a plurality of cylindrical slot portions such as four slots may be arranged on one circumference so that all the slot portions are integrally rotationally driven.

The opening 27 is formed to penetrate part of the bottom surface 26a of the cylindrical portion storage case 26, connects inside of part of the cylindrical portion 25 in the cylindrical portion storage case 26 and the processing tank 20, and allows the used paper diaper 18 in the cylindrical portion 25 to fall into the processing tank 20.

The cylindrical portion rotation drive unit 28 is, for example, a motor. A rotating shaft of the motor 28 is connected to a central shaft portion 25b of the cylindrical portion 25, and under the control of the control apparatus 11, the cylindrical portion 25 is rotated with respect to the cylindrical portion storage case 26 by rotational driving of the motor 28, so that the put-into slot 24 is sequentially positioned at the put-into position

I overlapping the opening 27.

The direct put-into door 21 and the storage put-into door 22 are attached to an upper portion of the cylindrical portion storage case 26 of the continuous put-into unit 23 so as to be able to be open and closed. The direct put-into door 21 is disposed so as to face the put-into slot 24 located at the put-into position I overlapping the opening 27. The storage put-into door 22 is disposed so as to face other put-into slots 24 located at the standby positions II other than the put-into position I overlapping the opening 27. Thus, the used paper diaper 18 put into from the direct put-into door 21 is directly put into the inner tank 5 through the opening 27. The used paper diaper 18 put into from the storage put-into door 22 is held in the standby slot 24b.

The outer tank 4 is a container capable of storing water. Water can be stored by being introduced into the processing tank 20 from the liquid supply unit 10, and a chemical can also be introduced into the processing tank 20 from the liquid supply unit 10. As a result, the processing liquid 19 can be supplied from the liquid supply unit 10 into the processing tank 20. Although described in detail later, the processing liquid 19 in which water and a chemical are mixed can also be supplied from the liquid supply unit 10 into the processing tank 20.

In addition, the liquid in the outer tank 4 is discharged to outside using the liquid discharge unit 9. Inside the outer tank 4, a cylindrical inner tank 5 having a plurality of through holes 5a on at least one of a side surface or a bottom surface is disposed. Each through hole 5a is smaller than the paper diaper 18, so that the paper diaper 18 does not fall off from the through hole 5a.

Further, the inner tank 5 is rotatably connected to the rotation drive unit 70. Under the control of the control apparatus 11, the rotation drive unit 70 rotatably drives the inner tank 5 at least two different types of rotational speed. As an example, the rotation drive unit 70 includes a shaft 13 fixed to the inner tank 5 along a rotation axis of the inner tank 5, a belt 14 that engages with the shaft 13 to rotate the shaft 13, and a motor 12 having a rotation shaft to which a gear engaged with the belt 14 is fixed. As a result, the inner tank 5 can be rotated with respect to the outer tank 4 by driving the motor 12 under the control of the control apparatus 11. As an example, the rotation axis of the inner tank 5 can be disposed in a vertical direction, or in an up-down direction including the vertical direction or a direction inclined more obliquely than the vertical direction.

For example, the rotation drive unit 70 performs the following operation under the control of the control apparatus 11.

The motor 12 rotates the inner tank 5 of the processing tank 20 to perform stirring processing to remove water from the used paper diaper 18.

After the stirring processing, the motor 12 rotates the inner tank 5 from which the processing liquid 19 has been discharged at higher speed than at the time of the stirring processing to perform dehydration processing.

The outer tank bottom portion 15 and the inner tank bottom portion 17 function as an example of an opening/closing unit and can be opened/closed around rotation axes by hinge portions 71 and 72 with respect to the outer tank 4 and the inner tank 5, respectively. The take-out unit 6 is disposed below the processing tank 20, that is, below the outer tank bottom portion 15 and the inner tank bottom portion 17 in the lower portion of the apparatus main body 3. The take-out unit 6 stores and holds the processed paper diaper 18 that falls from the inner tank 5 as a result of the outer tank bottom portion 15 and the inner tank bottom portion 17 being opened.

This enables the paper diaper after the weight reduction processing to be taken out from the take-out unit 6. The storage container 7 to which the disposal bag 8 is attached is disposed inside the take-out unit 6. The disposal bag 8 is disposed immediately below the inner tank bottom portion 17, and the paper diaper 18 after the weight reduction processing falls into the disposal bag 8 of the storage container 7.

### [Operation]

Next, the weight reduction processing operation of the used paper diaper processing apparatus 1 under the control of the control apparatus 11 will be described.

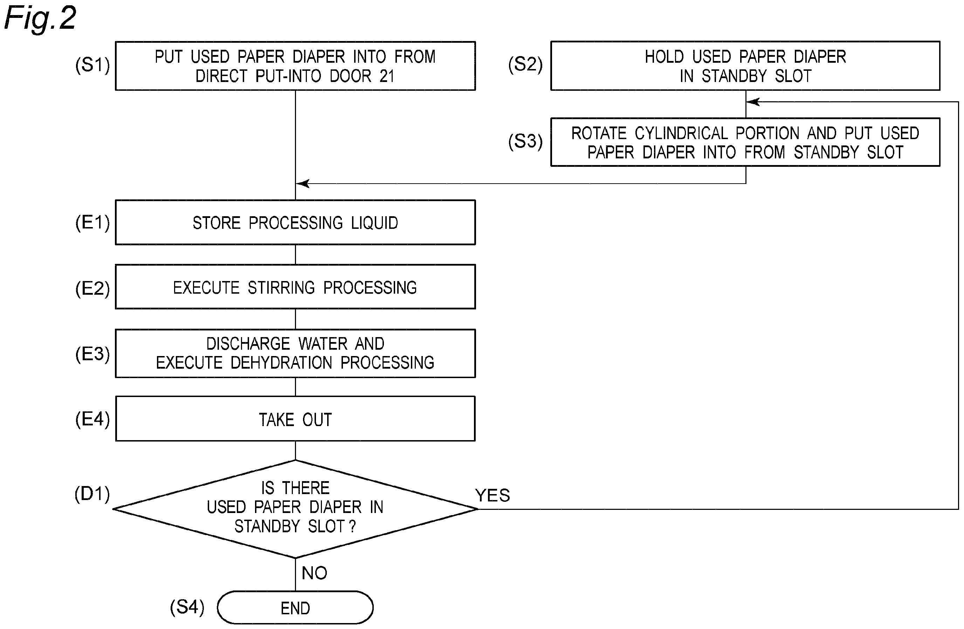

First, overall processing flow will be described with reference to Fig. 2.

In step S1, the used paper diaper 18 is put into the direct put-into slot 24a from the direct put-into door 21. The used paper diaper 18 put into the direct put-into slot 24a is stored in the inner tank 5 through the opening 27.

Next, in step S2, the used paper diaper 18 is held in the put-into slot 24. Note that if there is an empty space in the put-into slot 24, the used paper diaper 18 can be held in the put-into slot 24 at any time not only after step S1 but also before step S1.

Next, in step E1, under the control of the control apparatus 11, the processing liquid 19 for lowering the water absorption function of the superabsorbent polymer contained in the used paper diaper 18 is supplied from the liquid supply unit 10 into the processing tank 20 and stored. A chemical that lowers the water absorption function of the superabsorbent polymer may be poured in advance, and water may be supplied from the liquid supply unit 10 to mix the processing liquid 19 in the processing tank 20.

Next, in step E2, under the control of the control apparatus 11, the inner tank 5 of the processing tank 20 is rotated to perform stirring processing, and water is removed from the used paper diaper 18.

Next, in step E3, under the control of the control apparatus 11, the liquid accumulated in the outer tank 4 is discharged from the liquid discharge unit 9, and then the inner tank 5 is rotated at higher speed than that at the time of the stirring processing, thereby dehydrating the used paper diaper 18.

Next, in step E4, when the outer tank bottom portion 15 and the inner tank bottom portion 17 are opened, the used paper diapers 18 fall into the disposal bag 8 provided in the storage container 7.

Next, in step D1, the control apparatus 11 confirms whether or not the used paper diaper is held in the standby slot 24b. Whether or not the used paper diaper is held in the standby slot 24b may be confirmed by, for example, a sensor provided at each of the standby slots 24b. As an example of a sensor, a transmission sensor can be used. In other words, it is possible to detect light from a light projecting unit toward a light receiving unit of the transmission sensor on the basis of whether or not the light is blocked by the paper diaper 18 put in the standby slot 24b. In a case where the control apparatus 11 confirms that there is the used paper diaper 18 in any of the standby slots 24b, the processing proceeds to step S3.

In step S3, under the control of the control apparatus 11, the cylindrical portion drive unit 28 rotates and, for example, rotates the cylindrical portion 25 clockwise when viewed from above. When the standby slot 24b in which the used paper diaper 18 is stored comes to the put-into position I of the direct put-into slot 24a, the used paper diaper 18 falls through the opening 27 and is stored in the processing tank 20. Note that, in step D1, whether or not the used paper diaper 18 is present in the standby slot 24b at the standby position II is confirmed by detection by the sensor provided in each of the standby slots 24b. However, the control apparatus 11 may confirm that the used paper diaper 18 is present in the standby slot 24b at the standby position II by rotating the cylindrical portion 25 and detecting with the sensor provided at the opening 27 that the used paper diaper 18 has fallen. As an example of the sensor, a transmission sensor can be used. In other words, it is possible to detect light from a light projecting unit toward a light receiving unit of the transmission sensor on the basis of whether or not the light is blocked by the paper diaper 18 put in the opening 27.

By executing steps E1 to E4 again, the weight reduction processing of the used paper diaper 18 put into from the standby slot 24b is executed.

Then, step D1 is executed again.

In step D1, when the control apparatus 11 confirms that there is no used paper diaper in all the standby slots 24b at the standby position II, the processing proceeds to step S4, and the series of processing ends.

Next, details of each processing operation will be described.

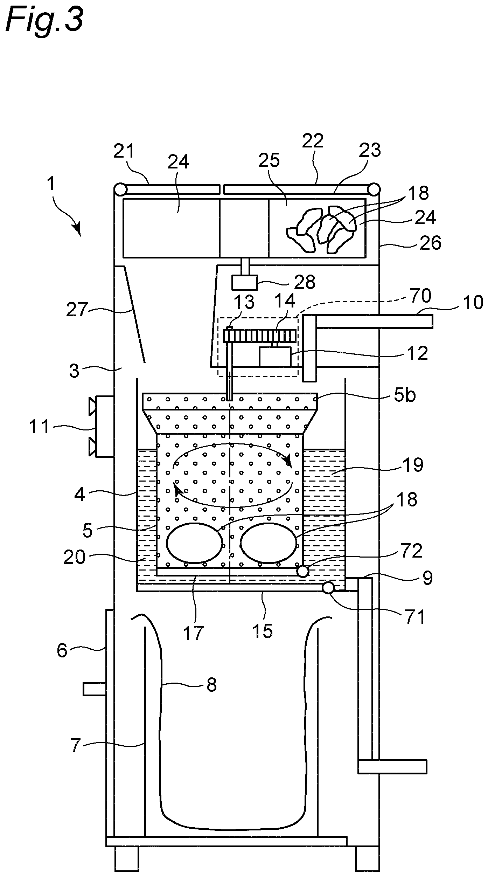

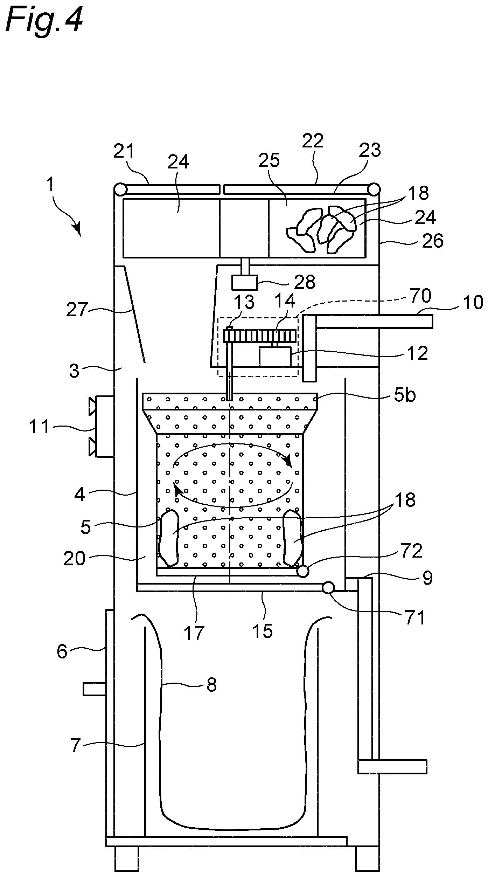

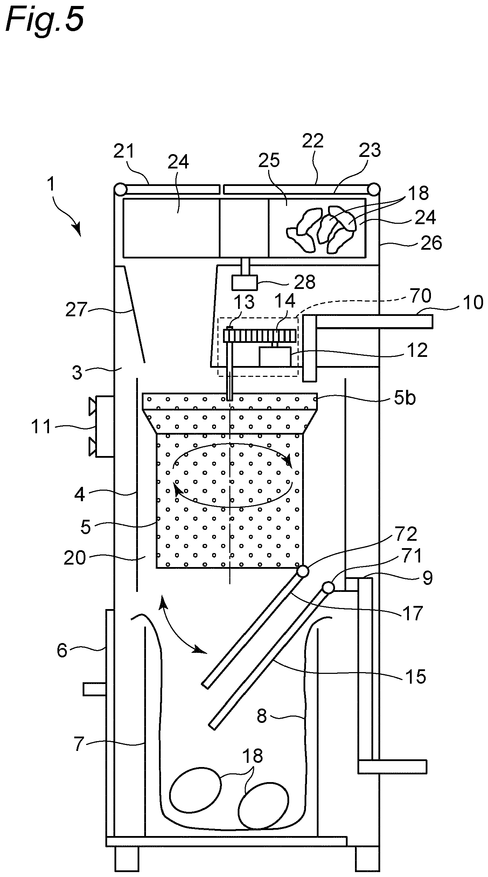

Hereinafter, for convenience of description, in the processing flow illustrated in Fig. 2, a state (Fig. 3) in which the stirring processing is performed in step E2 after step S1 and step S2 are completed and the used paper diaper 18 is put into the inner tank 5, a state (Fig. 4) in which the inner tank 5 is rotated at high speed to perform the dehydration processing in step E3, and a state (Fig. 5) in which the outer tank bottom portion 15 and the inner tank bottom portion 17 are opened in step E4 to perform the operation of taking out the used paper diaper 18 after the dehydration processing to the take-out unit 6 will be separately described.

First, in Fig. 3, after a chemical that lowers the water absorption function of the superabsorbent polymer is poured into the processing tank 20 from the liquid supply unit 10, supply of the processing liquid from the liquid supply unit 10 to the processing tank 20 is started, and the processing liquid 19 is stored in the processing tank 20 (see step E1). As an example of the chemical that lowers the water absorption function of the superabsorbent polymer, a chemical containing metal ions is used, and in particular, calcium chloride, lime, or the like, containing a divalent metal can be used.

Next, the motor 12 is rotationally controlled under the control of the control apparatus 11 to rotate the inner tank 5 at first rotation speed, thereby executing the stirring processing (see step E2). Specifically, when the motor 12 rotates at the first rotation speed, the shaft 13 rotates via the belt 14, and the inner tank 5 rotates with respect to the outer tank 4. As a result, the processing liquid 19 and the used paper diaper 18 are stirred. In this stirring processing, for example, it is possible that the inner tank 5 does not continue to rotate, but repeats after rotation for a certain period of time (for example, 3 to 5 seconds), stop, reverse rotation for a certain period of time (for example, 3 to 5 seconds), and stop, or repeats after rotation for a certain period of time (for example, 3 to 5 seconds), stop, and rotation for a certain period of time (for example, 3 to 5 seconds) in the same direction again, because stirring easily progresses with such repetition.

When the stirring processing is continued, the superabsorbent polymer of the used paper diaper 18 reacts with, for example, divalent metal ions in the chemical, so that water contained in the superabsorbent polymer is removed from the used paper diaper 18, and the removed water is mixed with the processing liquid 19. In the stirring processing here, it is sufficient that the processing liquid 19 and the superabsorbent polymer of the used paper diaper 18 react with each other, and it is important to gently stir under the control of the control apparatus 11 so that the used paper diaper 18 is not separated and disassembled by the stirring processing. Specifically, for example, it is possible that the first rotation speed is, for example, 40 to 160 rpm so that stirring is performed slowly.

Next, the dehydration processing of the used paper diaper 18 will be described with reference to Fig. 4 (see step E3). After the liquid such as the processing liquid accumulated in the outer tank 4 is discharged from the liquid discharge unit 9, the inner tank 5 is rotated at second rotation speed with respect to the outer tank 4 at high speed, so that the used paper diaper 18 can be dehydrated and the used paper diaper 18 can be reduced in weight. As a specific example of the second rotation speed, for example, it is possible to perform dehydration at rotation speed of about 800 to 1300 rpm.

Next, processing of taking out the used paper diaper 18 will be described with reference to Fig. 5 (see step E4). When the outer tank bottom portion 15 and the inner tank bottom portion 17 are opened via the hinge portions 71 and 72 in a state where the dehydration is completed, the used paper diaper 18 falls into the disposal bag 8 provided in the storage container 7. In a case where the used paper diaper 18 is discharged, it is possible to discharge the used paper diaper only by taking out the take-out unit 6 from the apparatus main body 3 by sliding the take-out unit 6 in a lateral direction thereof with respect to the apparatus main body 3, and taking out the disposal bag 8 from the storage container 7.

According to the first embodiment described above, when the weight reduction processing of the used paper diaper 18 put into the processing tank 20 is completed, the used paper diaper 18 held in the standby slot 24b is automatically put into the processing tank 20, and the weight reduction processing can be continuously performed, so that it is possible to perform the weight reduction processing of the used paper diaper 18 without a non-operating time of the apparatus.

In addition, the worker can cause the holding unit of the apparatus 1 to hold the used paper diaper 18 even in the middle of the weight reduction processing. Specifically, even when the used paper diaper processing apparatus 1 is in operation, it is possible to put the collected used paper diaper 18 into the standby slot 24b of the continuous put-into unit 23. It is therefore not necessary for the worker to start the next weight reduction processing at a timing when the weight reduction processing is completed, so that it is also possible to reduce the load on the worker for operating the apparatus 1. In other words, it is possible to efficiently perform the weight reduction processing of the used paper diaper 18 without bothering the worker.

In addition, the used paper diaper 18 subjected to the weight reduction processing with the chemical containing metal ions, or the like, has a fiber portion left without being separated and disassembled in the processing tank 20, and thus, it is not necessary to introduce an apparatus for removing fiber components of the used paper diaper 18 by a filter when liquid is discharged to a sewer pipe, or the like.

In addition, the worker does not have to directly take out the used paper diaper 18 after the processing from an upper side of the inner tank, and the processed paper diaper can be stored in the disposal bag 8 without bothering the worker, so that it is possible to reduce the work load and the hygiene load of the worker.

It is therefore possible to perform a series of operation such as weight reduction processing and storage of the used paper diaper 18 in the discharge container without bothering the worker.

Note that in the first embodiment, the disposal bag 8 for disposal is provided in the storage container 7, but the disposal bag 8 does not have to be provided and may be directly stored in the storage container 7.

In addition, instead of a case where the processing liquid 19 in which water and the chemical are mixed may be separately prepared and the prepared processing liquid 19 is directly stored in the processing tank 20, the chemical containing metal ions, or the like, may be poured into the processing tank 20, and then water may be stored to prepare the processing liquid 19 in the processing tank 20.

In order to rotate the inner tank 5, the rotating shaft of the motor 12 may be directly connected to the inner tank 5 without using the belt 14, so that the inner tank 5 can be directly rotationally driven by the motor 12.

### [Second Embodiment]

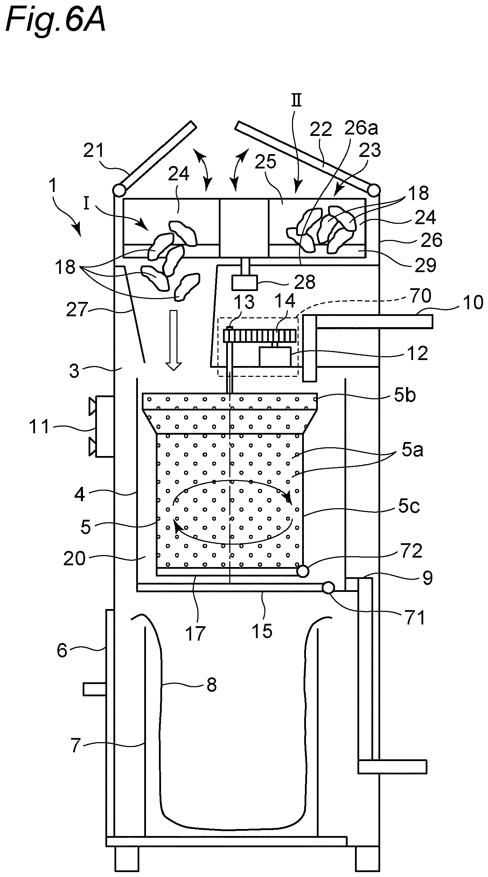

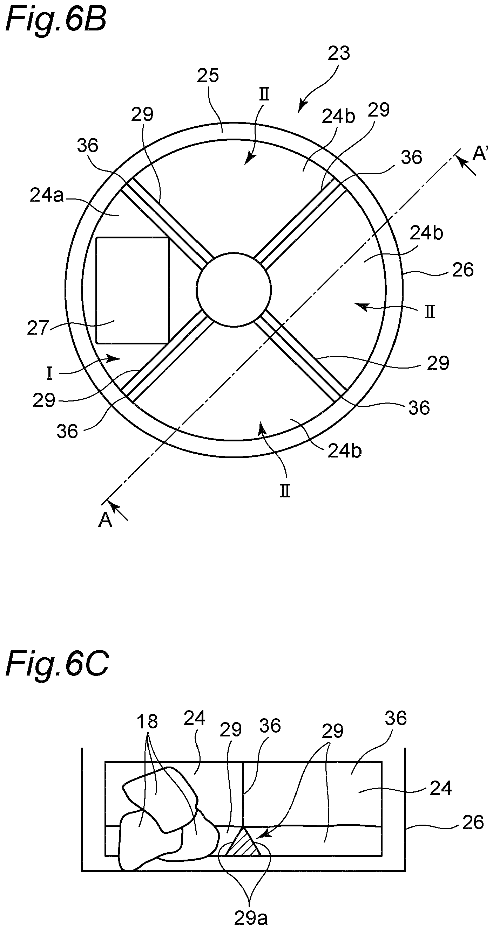

Figs. 6A to 6C illustrate an apparatus configuration of the second embodiment of the present disclosure. Fig. 6A illustrates an apparatus side view, Fig. 6B illustrates a top view of the cylindrical portion 25 and the cylindrical portion storage case 26 in a state where the direct put-into door 21 and the storage put-into door 22 are omitted, and Fig. 6C illustrates a cross-sectional view taken along line A-A' of Fig. 6B.

A difference from the first embodiment is that an anti-sandwiching portion 29 is attached to the lower portion of each partition plate 36 of the put-into slot 24 disposed inside the cylindrical portion 25.

The anti-sandwiching portion 29 is constituted with, for example, a triangular pyramid extending along a lower edge of each partition plate 36 and having an inclined surface 29a. The inclined surface 29a is, for example, disposed at least on a front side in a rotation direction of the cylindrical portion 25.

In Fig. 6C, when there is no anti-sandwiching portion 29, there is an issue that the used paper diaper 18 is sandwiched between the partition plate 36 and the cylindrical portion storage case 26 when the cylindrical portion 25 rotates to drop the used paper diaper 18 from the standby slot 24b into the processing tank 20.

On the other hand, according to the configuration of the second embodiment, the anti-sandwiching portion 29 is provided at the lower portion of the partition plate 36, and thus, the used paper diaper 18 is pushed upward from the cylindrical portion storage case 26 by the inclined surface 29a of the anti-sandwiching portion 29 at the time of rotation of the cylindrical portion 25, so that the cylindrical portion 25 can be rotated without the used paper diaper 18 being sandwiched between the partition plate 36 and the cylindrical portion storage case 26, and the used paper diaper 18 can be reliably and smoothly put into the processing tank 20.

According to the second embodiment described above, in addition to the operational effects of the first embodiment, when the cylindrical portion 25 rotates in the continuous put-into unit 23 to drop the used paper diaper 18 from the put-into slot 24 into the processing tank 20, the used paper diaper 18 is not sandwiched between the partition plate 36 and the cylindrical portion storage case 26, so that the weight reduction processing can be efficiently completed.

### [Third Embodiment]

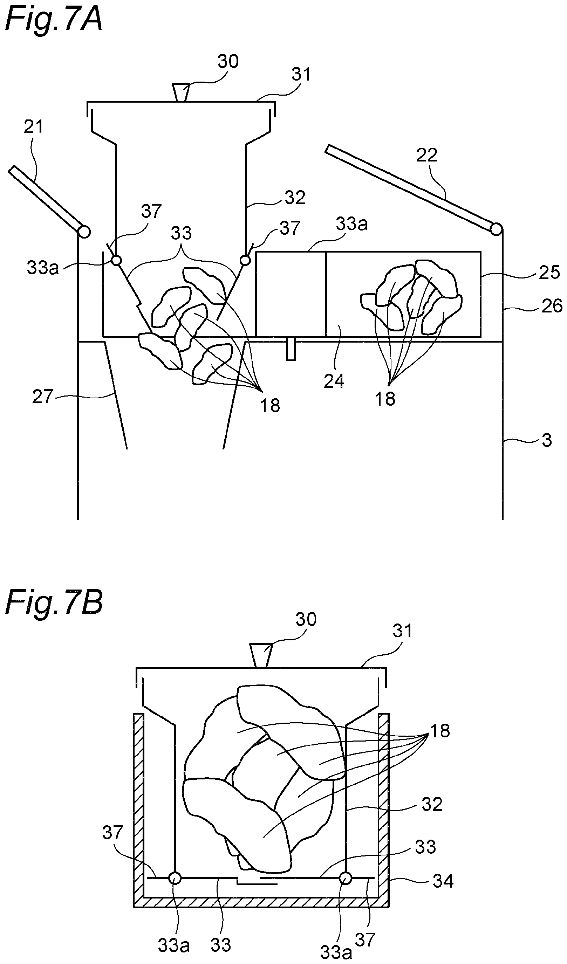

Figs. 7A and 7B illustrate an apparatus configuration of the third embodiment of the present disclosure. Fig. 7A illustrates an enlarged view of the continuous put-into unit 23 and illustrates a state in which the removable container 32 is positioned on top of the put-into slot 24. Fig. 7B illustrates a state in which the removable container 32 is provided on the table 34.

An upper end opening of the container 32 can be covered with a lid 31 with a handle 30. A lower part of the container 32 is divided into a plurality of parts (for example, left and right in Fig. 7B) and can be opened by container lower lids 33 that can be opened and closed by hinge portions 33a. In addition, a claw 37 that can be brought into contact with an upper end edge of a side wall of the cylindrical portion 25 that forms the put-into slot 24a is attached to an outer end portion on the hinge portion side of each container lower lid 33.

The container 32 can store a plurality of used paper diapers 18. When the container 32 is positioned above the put-into slot 24a and pushed downward from above, the claws 37 of the container lower lids 33 are caught by the upper end edge of the side wall of the cylindrical portion 25, and the container lower lids 33 rotate around the hinge portion 33a to open both the container lower lids 33. At this time, the used paper diaper 18 falls from a lower portion of the container 32 and is put into the inner tank 5.

By adopting such an apparatus configuration, it is possible to put the paper diaper 18 into the inner tank 5 without the worker directly touching the used paper diaper 18.

Further, the container 32 can be used as a normal trash box by being provided on the table 34 having an outer container shape. At this time, a size of the table 34 is set such that the claws 37 and the upper end edge of the table 34 do not come into contact with each other.

According to the configuration of the third embodiment, the container 32 provided on the table 34 is provided in a room in which the elderly live, so that it can be used as a trash box for discarding the paper diaper at the time of excretion assistance. Then, in a case where the weight reduction processing is performed, by pushing the container 32 into the standby slot 24, the worker can put the used paper diaper 18 into the paper diaper processing apparatus 1 without directly touching the used paper diaper 18.

As a result, it is possible to more efficiently perform the weight reduction processing on the used paper diaper 18.

According to the present third embodiment described above, in addition to the operational effects of the first embodiment, the used paper diaper 18 can be put into the paper diaper processing apparatus 1 by a simpler method, and the weight reduction processing can be completed more efficiently in a short period of time.

### [Fourth Embodiment]

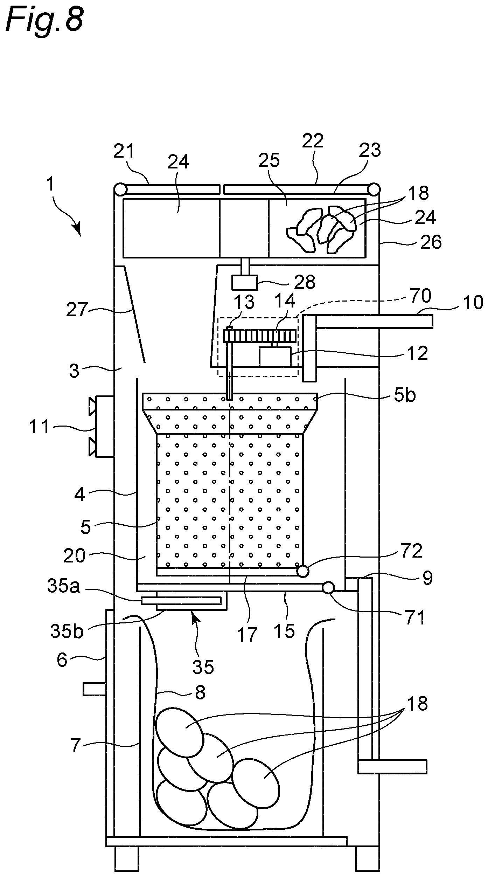

Fig. 8 illustrates an apparatus configuration of the fourth embodiment of the present disclosure.

A difference from the first embodiment is that a scraping portion 35 is attached to the lower surface of the outer tank bottom portion 15.

When the used paper diaper 18 is continuously subjected to the weight reduction processing, as illustrated in Fig. 8, the processed used paper diaper 18 is unevenly accumulated on a side on which the outer tank bottom portion 15 is opened in the disposal bag 8 of the take-out unit 6, which causes an issue that a volume of the storage container 7 provided in the lower portion cannot be effectively used.

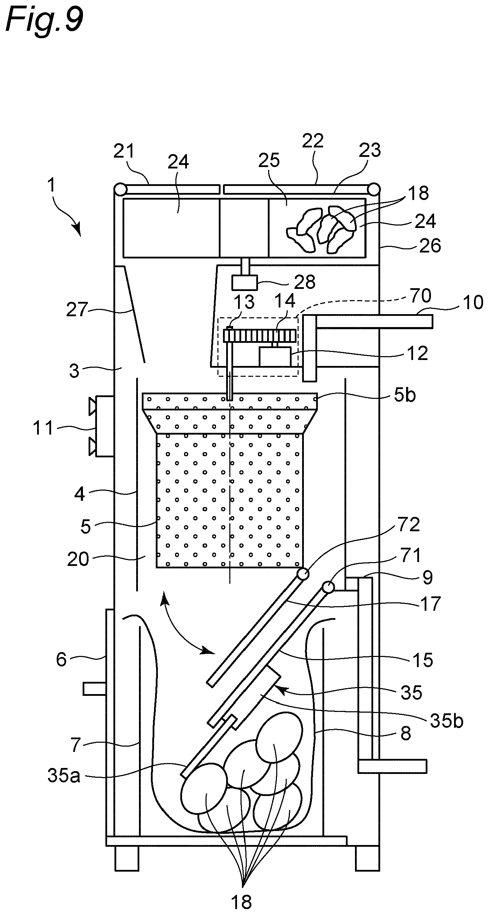

Fig. 9 illustrates a state in which the accumulated used paper diapers 18 that have been processed are scraped by the scraping portion 35, that is, moved to a side on which the user paper diapers 18 are not accumulated.

The scraping portion 35 attached to the outer tank bottom portion 15 has a structure in which a plate-shaped scraping member 35a can extend with respect to the storage case 35b in accordance with opening of the outer tank bottom portion 15. For example, it is possible to apply a structure in which, at a timing when the outer tank bottom portion 15 opens the outer tank 4, the scraping member 35a extends by the scraping member 35a causing a cylinder rod fixed at a distal end thereof to extend with respect to a cylinder which is the storage case 35b. The timing at which the outer tank bottom portion 15 opens the outer tank 4 can be detected by a known opening/closing detection switch provided on the outer tank bottom portion 15, or transition information to step E4 in the control apparatus 11 can be used. The present disclosure is not limited to such a cylinder mechanism and may have a structure using a cam.

By the scraping member 35a of the scraping portion 35 extending with respect to the storage case 35b in accordance with opening of the outer tank 4 by the outer tank bottom portion 15, it is possible to scrape the used paper diaper 18 to an opposite side in a left and right direction in Fig. 9 from a side where the used paper diapers have been accumulated.

According to the configuration of the present fourth embodiment, when the weight reduction processing is continuously performed on the used paper diaper 18, the weight reduction processing can be performed while scraping the processed used paper diaper 18, so that it is possible to solve an issue that the used paper diapers 18 are accumulated on one side of the storage container 7 and the volume of the storage container 7 cannot be effectively used, and it is possible to continuously perform the weight reduction processing more times. As a result, the work load of the worker can be reduced.

According to the fourth embodiment described above, in addition to the operational effects of the first embodiment, it is possible to continuously perform the weight reduction processing more times, and it is possible to reduce the non-operating time of the apparatus due to the storage container 7 being full, so that it is possible to more efficiently.perform the weight reduction processing.

Although the embodiments of the present disclosure have been described above, the above-described embodiments are merely examples for carrying out the present disclosure. Thus, the present disclosure is not limited to the above-described embodiments, and the above-described embodiments can be appropriately modified and implemented without departing from the gist thereof.

For example, by appropriately combining arbitrary embodiments or modified examples among the various embodiments or modified examples described above, it is possible to achieve the effects of the respective embodiments or modified examples. In addition, it is possible to combine embodiments, combine examples, or combine embodiments and examples, and it is possible to combine features in different embodiments or examples.

The used paper diaper processing apparatus according to the above aspect of the present disclosure can reduce weight of the used paper diaper by mixing the superabsorbent polymer that has absorbed water contained in excrement with the chemical containing divalent metal ions, or the like, to lower the water absorption function of the superabsorbent polymer to remove water and reducing an amount of water contained in the used paper diaper. In addition, the used paper diaper processing apparatus according to the above-described aspect of the present disclosure does not require the worker to directly take out the processed used paper diaper from the inner tank, so that it is possible to reduce the work load and the hygiene load of the worker, and it is possible to use the used paper diaper processing apparatus to improve work in elderly facilities that use a large amount of paper diapers or childcare facilities.

Although the present disclosure has been fully described in connection with the embodiments thereof with reference to the accompanying drawings, it is to be noted that various changes and modifications are apparent to those skilled in the art. Such changes and modifications are to be understood as included within the scope of the present disclosure as defined by the appended claims unless they depart therefrom.

## Claims

1. A used paper diaper processing apparatus (1) comprising:
a processing tank (20) that processes a used paper diaper (18);
a liquid supply unit (10) that supplies a processing liquid to the processing tank;
a liquid discharge unit (9) that discharges the processing liquid from the processing tank;
a take-out unit (6) that takes out and holds a paper diaper (18) processed in the processing tank below the processing tank;
the processing tank (20) including, on at least one of a side surface and a bottom surface, a cylindrical inner tank (5) having a plurality of through holes (5a) smaller than the paper diaper, and an outer tank (4) surrounding the side surface and the bottom surface of the inner tank;
a rotation drive unit (70) that rotates the inner tank (5) around an axis of the inner tank;
an opening/closing unit (15, 17) that discharges the paper diaper processed in the processing tank from a bottom portion (15) of the processing tank to the take-out unit (6);
said apparatus (1) being **characterized in that** it comprises
a continuous put-into unit (23) that includes in an upper portion of the processing tank, the continuous put-into unit including a plurality of holding units (24) each capable of holding a used paper diaper (18), and a holding unit rotation drive unit (28) that rotationally drives each of the holding units (24) at a put-into position (I) above an opening (27) connected to the processing tank (20) and a standby position (II) other than the put-into position,
wherein when the holding unit is rotated by the holding unit rotation drive unit (28) and positioned at the put-into position, the used paper diaper held by the holding unit is put into the processing tank from the opening.

2. The used paper diaper processing apparatus according to claim 1, wherein
the continuous put-into unit (23) includes a cylindrical portion (25) rotationally driven by the holding unit rotation drive unit (28), and
the plurality of holding units are constituted by partitioning the cylindrical portion with a partition plate (36), and an anti-sandwiching portion (29) having an inclined surface (29a) that lifts the paper diaper upward is provided at a lower portion of the partition plate.

3. The used paper diaper processing apparatus according to claim 1, further comprising:
a container (32) in which a side wall of the holding unit is brought into contact with a claw (37) at an outer end by being placed on the holding unit, and a container lower lid (33) is open around a hinge portion (33a)_{.}

4. The used paper diaper processing apparatus according to claim 2, further comprising:
a container (32) in which a side wall of the holding unit is brought into contact with a claw (37) at an outer end by being placed on the holding unit, and a container lower lid (33) is open around a hinge portion (33a).

5. The used paper diaper processing apparatus according to claim 1, further comprising:
a scraping portion (35) that is provided on a lower surface of a bottom portion (15) of the outer tank that is the opening/closing unit (15,17) so as to be able to extend and is capable of moving the processed paper diaper in the take-out unit when the opening/closing unit (15,17) is opened.

6. The used paper diaper processing apparatus according to claim 2, further comprising:
a scraping portion (35) that is provided on a lower surface of a bottom portion (15) of the outer tank that is the opening/closing unit (15,17) so as to be able to extend and is capable of moving the processed paper diaper (18) in the take-out unit (6) when the opening/closing unit is opened.

7. The used paper diaper processing apparatus according to claim 3, further comprising:
a scraping portion (35) that is provided on a lower surface of a bottom portion (15) of the outer tank that is the opening/closing unit (15,17) so as to be able to extend and is capable of moving the processed paper diaper (18) in the take-out unit (6) when the opening/closing unit is opened.

8. The used paper diaper processing apparatus according to claim 4, further comprising:
a scraping portion (35) that is provided on a lower surface of a bottom portion (15) of the outer tank that is the opening/closing unit (15,17) so as to be able to extend and is capable of moving the processed paper diaper (18) in the take-out unit (6) when the opening/closing unit is opened.

## Patentansprüche

1. Verarbeitungsvorrichtung (1) für benutzte Papierwindeln, mit:
einem Verarbeitungstank (20), der eine benutzte Papierwindel (18) verarbeitet,
einer Flüssigkeitszuführeinheit (10), die dem Verarbeitungstank eine Verarbeitungsflüssigkeit zuführt,
eine Flüssigkeitsablasseinheit (9), die die Verarbeitungsflüssigkeit aus dem Verarbeitungstank ablässt,
eine Herausnehmeinheit (6), die eine Papierwindel (18), die in dem Verarbeitungstank verarbeitet wurde, herausnimmt und unterhalb des Verarbeitungstanks hält,
wobei der Verarbeitungstank (20) an einer Seitenfläche und/oder einer Bodenfläche einen zylindrischen inneren Tank (5) mit mehreren Durchgangslöchern (5a), die kleiner als die Papierwindel sind, und einen äußeren Tank (4) aufweist, der die Seitenfläche und die Bodenfläche des inneren Tanks umgibt,
eine Rotationsantriebseinheit (70), die den inneren Tank (5) um eine Achse des inneren Tanks rotiert,
eine Öffnungs-/Schließ-Einheit (15, 17), die die Papierwindel, die in dem Verarbeitungstank verarbeitet wurde, aus einem Bodenabschnitt (15) des Verarbeitungstanks in die Herausnehmeinheit (6) entlädt,
wobei die Vorrichtung (1) **dadurch gekennzeichnet ist, dass** sie eine Einheit (23) für eine kontinuierliche Eingabe aufweist, die in einem oberen Abschnitt des Verarbeitungstanks enthalten ist, wobei die Einheit für die kontinuierliche Eingabe mehrere Halteeinheiten (24), die jeweils dazu in der Lage sind, eine benutzte Papierwindel (18) zu halten, und eine Halteeinheitsrotationsantriebseinheit (28) umfasst, die in rotierender Weise jede der Halteeinheiten (24) an einer Eingabeposition oberhalb einer Öffnung (27), die mit dem Verarbeitungstank (20) verbunden ist, und an einer von der Eingabeposition abweichenden Bereitschaftsposition (II) antreibt,
wobei, wenn die Halteeinheit durch die Halteeinheitsrotationsantriebseinheit (28) rotiert und an der Eingabeposition positioniert wird, die benutzte Papierwindel, die durch die Halteeinheit gehalten wird, von der Öffnung in den Verarbeitungstank eingegeben wird.

2. Verarbeitungsvorrichtung für benutzte Papierwindeln nach Anspruch 1, wobei
die Einheit (23) für die kontinuierliche Eingabe einen zylindrischen Abschnitt (25) aufweist, der rotierend durch die Halteeinheitsrotationsantriebseinheit (28) angetrieben wird, und
die mehreren Halteeinheiten durch Unterteilen des zylindrischen Abschnitts mit einer Unterteilungsplatte (36) gebildet sind und ein Anti-Sandwiching-Abschnitt (29) mit einer geneigten Oberfläche (29a), die die Papierwindel anhebt, an einem unteren Abschnitt der Unterteilungsplatte vorgesehen ist.

3. Verarbeitungsvorrichtung für benutzte Papierwindeln nach Anspruch 1, ferner mit:
einem Container (32), in dem eine Seitenwandung der Halteeinheit in Kontakt mit einer Klaue (37) an einem äußeren Ende gebracht wird, indem sie auf der Halteeinheit platziert wird, und ein unterer Containerdeckel (33) um einen Scharnierabschnitt (33a) offen ist.

4. Verarbeitungsvorrichtung für benutzte Papierwindeln nach Anspruch 2, ferner mit:
einem Container (32), in dem eine Seitenwandung der Halteeinheit in Kontakt mit einer Klaue (37) an einem äußeren Ende gebracht wird, indem sie auf der Halteeinheit platziert wird, und ein unterer Containerdeckel (33) um einen Scharnierabschnitt (33a) offen ist.

5. Verarbeitungsvorrichtung für benutzte Papierwindeln nach Anspruch 1, ferner mit:
einem Kratzabschnitt (35), der an einer unteren Oberfläche eines Bodenabschnitts (15) des äußeren Tanks, der die Öffnungs-/Schließ-Einheit (15, 17) ist, vorgesehen ist, so dass er dazu in der Lage ist, sich auszudehnen, und dazu ausgestaltet ist, die verarbeitete Papierwindel in die Herausnehmeinheit zu bewegen, wenn die Öffnungs-/SchließEinheit (15, 17) geöffnet ist.

6. Verarbeitungsvorrichtung für benutzte Papierwindel nach Anspruch 2, ferner mit:
einem Kratzabschnitt (35), der an einer unteren Oberfläche eines Bodenabschnitts (15) des äußeren Tanks, der die Öffnungs-/Schließ-Einheit (15, 17) ist, vorgesehen ist, so dass er dazu in der Lage ist, sich auszudehnen, und dazu ausgestaltet ist, die verarbeitete Papierwindel (18) in die Herausnehmeinheit (6) zu bewegen, wenn die Öffnungs-/Schließ-Einheit geöffnet ist.

7. Verarbeitungsvorrichtung für benutzte Papierwindeln nach Anspruch 3, ferner mit:
einem Kratzabschnitt (35), der an einer unteren Oberfläche eines Bodenabschnitts (15) des äußeren Tanks, der die Öffnungs-/Schließ-Einheit (15, 17) ist, vorgesehen ist, so dass er dazu in der Lage ist, sich auszudehnen, und dazu ausgestaltet ist, die verarbeitete Papierwindel (18) in die Herausnehmeinheit (6) zu bewegen, wenn die Öffnungs-/Schließ-Einheit geöffnet ist.

8. Verarbeitungsvorrichtung für benutzte Papierwindeln nach Anspruch 4, ferner mit:
einem Kratzabschnitt (35), der an einer unteren Oberfläche eines Bodenabschnitts (15) des äußeren Tanks, der die Öffnungs-/Schließ-Einheit (15, 17) ist, vorgesehen ist, so dass er dazu in der Lage ist, sich auszudehnen, und dazu ausgestaltet ist, die verarbeitete Papierwindel (18) in die Herausnehmeinheit (6) zu bewegen, wenn die Öffnungs-/Schließ-Einheit geöffnet ist.

## Revendications

1. Dispositif de traitement (1) de couches en papier usagées, comprenant:
un réservoir de traitement (20) qui traite une couche en papier usagée (18),
une unité d'alimentation en liquide (10) qui fournit un liquide de traitement au réservoir de traitement,
une unité de décharge de liquide (9) qui décharge le liquide de traitement du réservoir de traitement,
une unité d'extraction (6) qui extrait une couche en papier (18) traitée dans le réservoir de traitement et maintient celle-ci au-dessous du réservoir de traitement,
le réservoir de traitement (20) comprenant, sur une surface latérale et/ou une surface de fond, un réservoir intérieur cylindrique (5) ayant une pluralité de trous traversants (5a) qui sont plus petits que la couche en papier, et un réservoir extérieur (4) entourant la surface latérale et la surface de fond du réservoir intérieur,
une unité d'entraînement en rotation (70) qui fait tourner le réservoir intérieur (5) autour d'un axe du réservoir intérieur,
une unité d'ouverture/de fermeture (15, 17) qui décharge la couche en papier traitée dans le réservoir de traitement, depuis une portion de fond (15) du réservoir de traitement vers l'unité d'extraction (6),
ledit dispositif (1) étant **caractérisé par le fait qu'**il comprend une unité d'introduction en continu (23) qui est comprise dans une portion supérieure du réservoir de traitement, l'unité d'introduction en continu comprenant une pluralité d'unités de maintien (24) qui sont chacune capable de maintenir une couche en papier usagée (18), et une unité d'entraînement en rotation d'unité de maintien (28) qui entraîne en rotation chacune des unités de maintien (24) sur une position d'introduction au-dessus d'une ouverture (27) reliée au réservoir de traitement (20) et une position d'attente (II) autre que la position d'introduction,
dans lequel, lorsque l'unité de maintien est tournée par l'unité d'entraînement en rotation d'unité de maintien (28) et est positionnée sur la position d'introduction, la couche en papier usagée maintenue par l'unité de maintien est introduite dans le réservoir de traitement depuis l'ouverture.

2. Dispositif de traitement de couches en papier usagées selon la revendication 1, dans lequel
l'unité d'introduction en continu (23) comprend une portion cylindrique (25) entraînée en rotation par l'unité d'entraînement en rotation d'unité de maintien (28), et
la pluralité d'unités de maintien sont constituées en divisant la portion cylindrique avec une plaque de séparation (36), et une portion anti-sandwich (29) ayant une surface inclinée (29a) qui soulève la couche en papier est prévue sur une portion inférieure de la plaque de séparation.

3. Dispositif de traitement de couches en papier usagées selon la revendication 1, comprenant en outre:
un conteneur (32) dans lequel une paroi latérale de l'unité de maintien est mise en contact avec une griffe (37) à une extrémité extérieure en étant placée sur l'unité de maintien, et un couvercle inférieur de conteneur (33) est ouvert autour d'une portion à charnière (33a).

4. Dispositif de traitement de couches en papier usagées selon la revendication 2, comprenant en outre:
un conteneur (32) dans lequel une paroi latérale de l'unité de maintien est mise en contact avec une griffe (37) à une extrémité extérieure en étant placée sur l'unité de maintien, et un couvercle inférieur de conteneur (33) est ouvert autour d'une portion à charnière (33a).

5. Dispositif de traitement de couches en papier usagées selon la revendication 1, comprenant en outre:
une portion de raclage (35) qui est prévue sur une surface inférieure d'une portion de fond (15) du réservoir extérieur, qui est l'unité d'ouverture/de fermeture (15, 17), de manière à être apte à s'étendre, et qui est capable de déplacer la couche traitée en papier dans l'unité d'extraction lorsque l'unité d'ouverture/de fermeture (15, 17) est ouverte.

6. Dispositif de traitement de couches en papier usagées selon la revendication 2, comprenant en outre:
une portion de raclage (35) qui est prévue sur une surface inférieure d'une portion de fond (15) du réservoir extérieur, qui est l'unité d'ouverture/de fermeture (15, 17), de manière à être apte à s'étendre, et qui est capable de déplacer la couche traitée en papier (18) dans l'unité d'extraction (6) lorsque l'unité d'ouverture/de fermeture est ouverte.

7. Dispositif de traitement de couches en papier usagées selon la revendication 3, comprenant en outre:
une portion de raclage (35) qui est prévue sur une surface inférieure d'une portion de fond (15) du réservoir extérieur, qui est l'unité d'ouverture/de fermeture (15, 17), de manière à être apte à s'étendre, et qui est capable de déplacer la couche traitée en papier (18) dans l'unité d'extraction (6) lorsque l'unité d'ouverture/de fermeture est ouverte.

8. Dispositif de traitement de couches en papier usagées selon la revendication 4, comprenant en outre:
une portion de raclage (35) qui est prévue sur une surface inférieure d'une portion de fond (15) du réservoir extérieur, qui est l'unité d'ouverture/de fermeture (15, 17), de manière à être apte à s'étendre, et qui est capable de déplacer la couche traitée en papier (18) dans l'unité d'extraction (6) lorsque l'unité d'ouverture/de fermeture est ouverte.
